# EUROPEAN PATENT APPLICATION

(11) **EP 4 848 033 A1**
(43) Date of publication of application: **02.12.2026**
(21) Application number: 26179834.2
(22) Date of filing: 20.05.2026
(51) Int. Cl.: G06F 1/16, A61B 5/00, A61B 5/11, G06F 1/3231, G06F 3/01, H04M 1/72454, H04M 1/60

(54) **SENSOR GATING BASED ON MOTION AND AUDIO WITH TIME-STAMPING**

(30) Priority: 27.05.2025 US 202563812414 P; 06.05.2026 US 202619669214
(71) Applicant: Apple Inc., Cupertino, CA 95014 (US)
(72) Inventor: CHIMALAMARRI, Anshu K., Cupertino, 95014 (US); RASTOGI, Manu, Cupertino, 95014 (US); WANG, Qian, Cupertino, 95014 (US); VADLAPUDI, Ravikiran, Cupertino, 95014 (US); AGARWAL, Sachin, Cupertino, 95014 (US)
(74) Representative: Zacco Denmark A/S

(57) **Abstract**

Various implementations disclosed herein include devices, systems, and methods that provide user experiences on a wearable device based on using sensors and processing capabilities to understand what the user is doing throughout an extended timeframe. For example, a process may include monitoring for changes in user activity during time span. Monitoring for changes in user activity may include: obtaining first and second sensor data from sensors in a physical environment in which a user is wearing the wearable device; synchronizing the first and second sensor data by identifying a first data element of the first sensor data and a second data element of the second sensor data occurring during a time segment; and detecting a change in activity of the user based on the synchronizing. The process may further identify a current activity of the user based on detecting the change in activity of the user.

## Description

### TECHNICAL FIELD

The present disclosure generally relates to systems, methods, and devices that provide user experiences on a wearable device based on a detected change in user activity.

### BACKGROUND

Existing techniques for efficiently providing user experiences via a wearable device may be improved with respect to accuracy, compute requirements and power consumption.

### SUMMARY

Various implementations disclosed herein include systems, methods, and devices that provide user experiences on a wearable device (e.g., an HMD, an audio headset, etc.) based on optimizing sensor usage and device processing capabilities to understand what a user is doing throughout an extended timeframe. For example, rather than capturing images constantly, the wearable device may be configured to detect a change in user activity (e.g., a context change) by monitoring low-power sensor data (e.g., inertial measurement unit (IMU) and audio data) and triggering or limiting the capture of images to select times such as, for example, subsequent to a detected user activity change. In some implementations, the captured images may be processed via compute/resource intensive processes to identify a user's new activity. For example, compute/resource intensive processes may include large language models (LLMs), etc.

In some implementations, audio, IMU and/or other low power sensor data may be synchronized using time stamps to create time stamped sensor data. The time-stamped sensor data may be considered in blocks of time. In some implementations, time-stamped sensor data may be input periodically into a data extractor (e.g., extracting a feature every 2 seconds) to extract features to be aggregated for a block of time (e.g., a last 5 periods corresponding to 10 seconds). In some implementations, two levels of information may be analyzed, concatenated and fed into a gating process to limit image capture. For example, the two levels of information may include a direct output from the feature extractor and an output from another process at a higher semantic level to provide a user activity prediction and associated probability distribution.

In some implementations, a wearable device has a processor (e.g., one or more processors) that executes instructions stored in a non-transitory computer-readable medium to perform a method. The method performs one or more steps or processes. In some implementations, the wearable device performs a first process during use of the wearable device over a time span to monitor for changes in user activity during the time span. The first process includes obtaining first sensor data and second sensor data from one or more sensors in a physical environment in which a user is wearing the wearable device; synchronizing the first sensor data with the second sensor data by identifying a first data element of the first sensor data and a second data element of the second sensor data occurring during a time segment; and detecting a change in activity of the user based on the synchronizing. In some implementations, the wearable device initiates a second process to identify a current activity of the user based on detecting the change in activity of the user.

In accordance with some implementations, a device includes one or more processors, a non-transitory memory, and one or more programs; the one or more programs are stored in the non-transitory memory and configured to be executed by the one or more processors and the one or more programs include instructions for performing or causing performance of any of the methods described herein. In accordance with some implementations, a non-transitory computer readable storage medium has stored therein instructions, which, when executed by one or more processors of a device, cause the device to perform or cause performance of any of the methods described herein. In accordance with some implementations, a device includes: one or more processors, a non-transitory memory, and means for performing or causing performance of any of the methods described herein.

### BRIEF DESCRIPTION OF THE DRAWINGS

So that the present disclosure can be understood by those of ordinary skill in the art, a more detailed description may be had by reference to aspects of some illustrative implementations, some of which are shown in the accompanying drawings.
Figures 1A and 1B illustrate exemplary electronic devices operating in a physical environment, in accordance with some implementations.
Figure 2 illustrates a view of a sensor gating framework configured to enable IMU and audio gating models to limit image capture during user activity detection, in accordance with some implementations.
Figure 3 illustrates a time-series dataset representing gating model context change detection based on IMU and audio features, in accordance with some implementations.
Figures 4A-4C illustrate views of a process implemented by the sensor gating framework of Figure 2 for detecting transitions in user activity of a user wearing a wearable device in a physical environment, in accordance with some implementations.
Figure 5 illustrates a system that leverages low power sensor data from IMU and audio sensors to track and predict user activities over extended periods without continuously capturing image data, in accordance with some implementations.
Figure 6 is a block diagram of an example system illustrating a user device configured to detect a change in user activity, in accordance with some implementations.
Figure 7 is a flowchart representation of an exemplary method that detects a change in user activity by monitoring low-power sensor data and triggers the capture of images at select times for processing to identify the user's new activity, in accordance with some implementations.
Figure 8 is an example electronic device in accordance with some implementations.

In accordance with common practice the various features illustrated in the drawings may not be drawn to scale. Accordingly, the dimensions of the various features may be arbitrarily expanded or reduced for clarity. In addition, some of the drawings may not depict all of the components of a given system, method or device. Finally, like reference numerals may be used to denote like features throughout the specification and Figures.

### DESCRIPTION

Numerous details are described in order to provide a thorough understanding of the example implementations shown in the drawings. However, the drawings merely show some example aspects of the present disclosure and are therefore not to be considered limiting. Those of ordinary skill in the art will appreciate that other effective aspects and/or variants do not include all of the specific details described herein. Moreover, well-known systems, methods, components, devices and circuits have not been described in exhaustive detail so as not to obscure more pertinent aspects of the example implementations described herein.

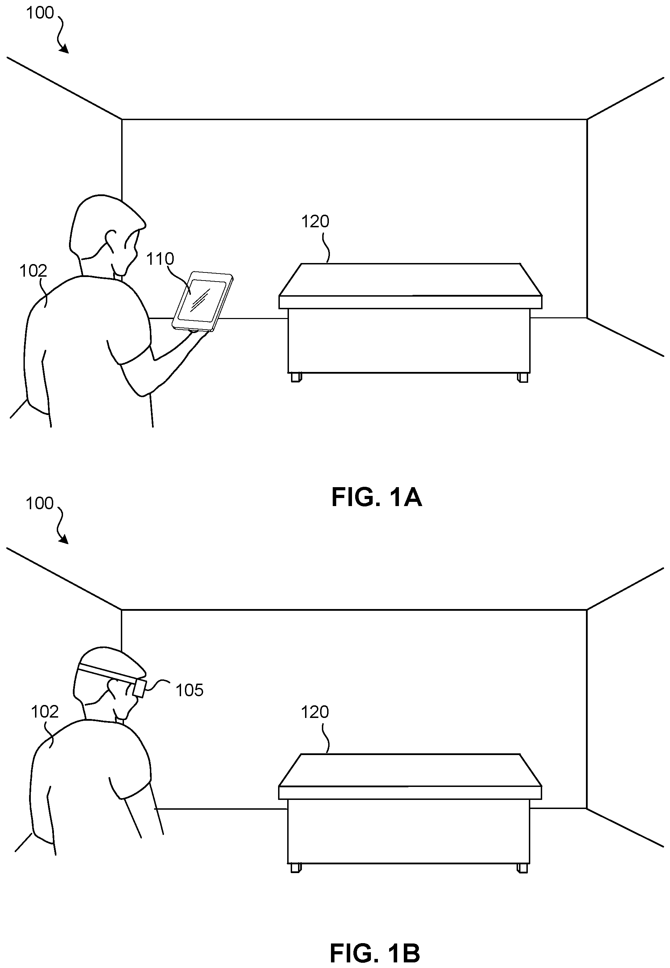

**Figures 1A-B** illustrate exemplary electronic devices 105 and 110 operating in a physical environment 100. In the example of Figures 1A-B, the physical environment 100 is a room that includes a desk 120. The electronic devices 105 and 110 may include one or more cameras, microphones, depth sensors, IMU sensors, or other sensors that can be used to capture information about and evaluate the physical environment 100 and the objects within it, as well as information about the user 102 of electronic devices 105 and 110. The information about the physical environment 100 and/or user 102 may be used to provide visual and audio content and/or to identify the current location of the physical environment 100 and/or the location of the user within the physical environment 100.

In some implementations, views of an XR environment may be provided to one or more participants (e.g., user 102 and/or other participants not shown) via electronic devices 105 (e.g., a wearable device such as an HMD or headphones) and/or 110 (e.g., a handheld device such as a mobile device, a tablet computing device, a laptop computer, etc.). Such an XR environment may include views of a 3D environment that is generated based on camera images and/or depth camera images of the physical environment 100 as well as a representation of user 102 based on camera images and/or depth camera images of the user 102. Such an XR environment may include virtual content that is positioned at 3D locations relative to a 3D coordinate system associated with the XR environment, which may correspond to a 3D coordinate system of the physical environment 100.

Various implementations disclosed herein include devices, systems, and methods that implement gaze tracking approaches that use image data. In some implementations, gaze may be tracked using imaging data to determine eye position or eye orientation using a pupil plus glint model, using a depth camera (e.g., stereo, structured light projection, time-of-flight (ToF), etc.) with 3D point cloud registration, or using an appearance-based model.

In some implementations, electronic devices 105 and 110 may enable an efficient pipeline for optimizing sensor usage and balancing power efficiency to detect a change in user activity (e.g., a context change). For example, a first process that leverages low-power sensors such as IMU sensors (e.g., accelerometer, gyroscope, magnetometer, etc.) and audio sensors to detect context changes may be used to enable a second process to dynamically trigger higher-power processes (e.g., image capture and LLM-based image/activity analysis) only when necessary. This two-level approach is configured to enable efficient identification of a current user activity (e.g., of user 102).

In some implementations, the first process may use IMU data (e.g., acceleration, angular velocity, etc.) to enable detection of transitions in user movement such as, for example, sitting to standing, standing to walking, head movements, etc. Likewise, the first process may additionally use audio data to complement the IMU data to further enable detection of transitions in movement by identifying environmental cues such as, for example, speech, background noise shifts, etc.

In some implementations, the IMU and audio data may be synchronized using timestamps to ensure coherent feature extraction and temporal aggregation. For example, using a feature extractor to enable periodic feature extraction (e.g., every 2 seconds) may help to maintain responsiveness. In some implementations, the extracted features may be aggregated over a longer block (e.g., 10 seconds) to smooth transient noise while preserving trends in user activity.

In some implementations, output from the feature extractor may provide immediate, low-latency signals. Likewise, output from a higher-level activity prediction model (e.g., a transformer-based model) may be used to refine a context understanding.

In some implementations, the aforementioned outputs may be concatenated and fed into a gating mechanism to limit unnecessary image capture thereby preserving power and bandwidth. In some implementations, different user activities may require different image capture frequencies (e.g., rapid bursts for dynamic activities, sparse sampling for static states, etc.) and therefore the pipeline may be configured to dynamically adjust image capture rates based on predicted activity confidence levels.

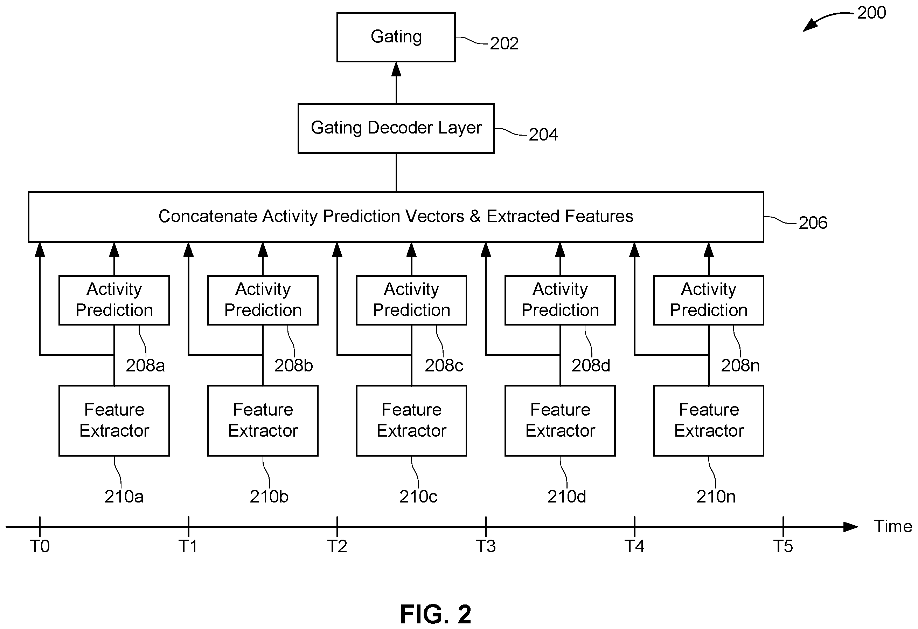

**Figure 2** illustrates a view of a sensor gating framework 200 configured to enable IMU and audio gating models to limit image capture (e.g., via a gating module 202) during user activity detection, in accordance with some implementations. In some implementations, an IMU gating model may be configured to continuously monitor IMU data (e.g., accelerometer data, gyroscope data, etc.) and detect transitions between user activities such as, for example, sitting to standing, standing to walking, vacuuming to exercise, etc. In some implementations, an IMU gating model may use threshold-based triggers, rule based algorithms, or machine learning (ML) models (e.g., a transformer) to detect motion transitions and when a motion transition is detected, the IMU gating model may be configured to signal an image capture system.

In some implementations, an audio gating model may be configured to run a low-power classifier with respect to ambient sounds (e.g., a vacuum motor hum, weights clanking, talking, etc.) to detect environmental/audio changes (e.g., a vacuum motor stops, music starts for workout, etc.). In this instance, the audio gating model may be configured to analyze the audio changes with respect to a frequency and time and/or pre-trained audio embeddings. If an audio change is detected, the audio gating model may be configured to additionally signal the image capture system.

In some implementations, the IMU and audio gating models may be configured to trigger an image capture sequence. For example, if a user activity transitions from vacuuming to exercise, an IMU gating model may detect a movement change (e.g., faster, varied motion) and an audio gating model may detect an audio shift (e.g., vacuum off, music on, etc.) and therefore both the IMU and audio gating models may be configured to confirm a context change thereby triggering an image capture via gating module 202. In another example, if a user activity transitions from sitting to standing to walking, an IMU gating model may detect a user postural change and an audio gating model may not detect any change as an audio signal may remain mostly unchanged during this user activity transition. Accordingly, IMU gating model may be configured to independently trigger a proactive image capture. Therefore, sensor gating framework 200 is configured to trigger capture of images (via gating module 202) only at key user activity transitions thereby preventing unnecessary image capture. Likewise, sensor gating framework 200 may be configured to provide descriptive metadata (e.g., vacuuming most likely stopped, possible user workout, etc.) enabled to initiate a higher-level ML model (e.g., LLM) to capture and process an image more efficiently.

In some implementations, sensor gating framework 200 comprises a hierarchical decision-making system that integrates low-level feature extraction (e.g., via feature extractor modules 210a...210n) with high-level activity prediction (e.g., via activity prediction modules 208a...208n) to control image capture. For example, sensor gating framework 200 enables a two-level feature aggregation approach to generate a gating signal for gating module 202. In some implementations, feature extractor modules 210a...210n are configured to extract (raw) features from non-vision signals (e.g., audio waveforms, IMU signals, etc.) and process associated data at fixed intervals T0-T5 (e.g., every 2 seconds). In some implementations, each of feature extractor modules 210a-210n is specific to one type of feature from non-vision signals (e.g., audio waveforms, IMU signals, etc.). For example, feature extractor module 210a may be specific to audio, feature extractor module 210b may be specific to IMU, etc. In some implementations, each of feature extractor modules 210a-210n may be inclusive of all different types of features from non-vision signals (e.g., audio waveforms, IMU signals, etc.). For example, each of feature extractor modules 210a-210n may be inclusive of audio waveforms, IMU signals, etc. Audio features may include, *inter alia,* spectrograms, energy levels, frequency bands, etc. IMU features may include, *inter alia,* acceleration patterns, angular velocity, movement frequency, etc. In some implementations, time stamps may be used to aggregate historical data.

In some implementations, activity prediction modules 208a...208n are configured to run an ML-based classifier and/or rule-based algorithm that maps extracted features to activity labels (e.g., walking, vacuuming, exercising, etc.) and output a probability distribution over activity classes thereby providing semantic context extending beyond raw feature trends. For example, rule based approaches may include filtering or threshold techniques configured to classify or infer user activity from features such as audio waveforms, IMU signals, etc.

In some implementations, sensor gating framework 200 includes a module 206 configured to concatenate feature information obtained from feature extractor modules 210a...210n and activity information obtained from prediction modules 208a...208n to combine the extracted low-level features with high-level activity predictions into a single feature vector. This concatenation process ensures that a gating decision is based on both real-time movement, audio signatures and a contextual understanding of a user's activity.

In some implementations, sensor gating framework 200 includes a gating decoder layer 204 configured to use binary classification to decide whether a current window signals a context change. For example, "1" may trigger image capture when a significant context change is detected and "0" may instruct no image capture if a user activity remains unchanged. In some implementations, the gating decoder layer 204 may be trained on large datasets to improve decision accuracy. In some implementations, the gating decoder layer 204 may be trained on historical sensor data to recognize meaningful transitions and use interval-based measurements to refine gating thresholds dynamically. Gating thresholds may include adaptive thresholds adjustable based on user movement trends and may use personalized models to tune based on individual behavior patterns.

Accordingly, sensor gating framework 200 improves power efficiency by avoiding unnecessary image captures. Likewise, sensor gating framework 200 improves context awareness by using both raw signals and semantic activity understanding.

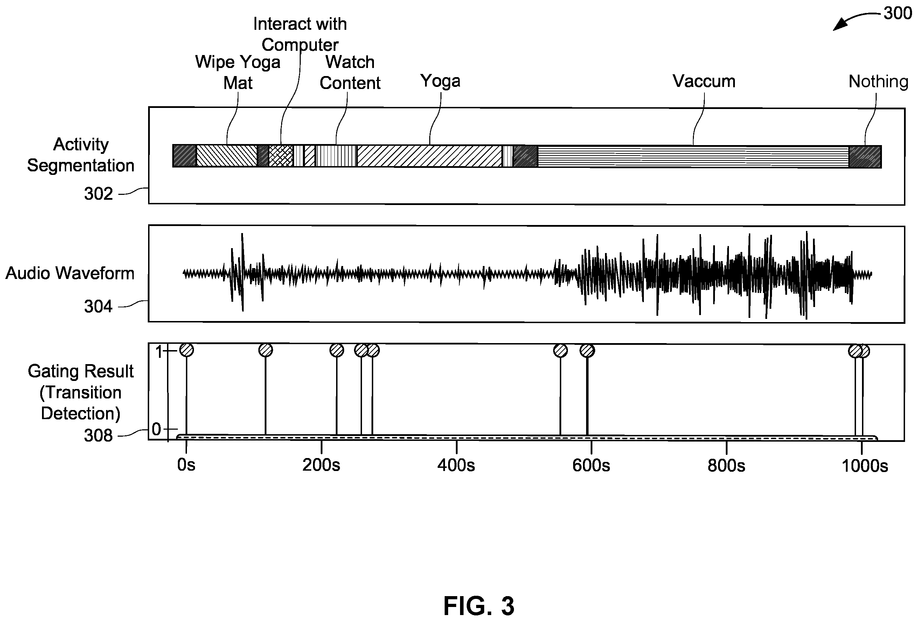

**Figure 3** illustrates a time-series dataset 300 representing gating model context change detection based on IMU and audio features, in accordance with some implementations. Dataset 300 includes activity segmentation data 302, audio waveform data 304, and gating result data 308.

In some implementations, time series-dataset 300 illustrates a user moving through different activities over time. For example, activity segmentation data 302 represents motion-related data (e.g., acceleration, angular velocity, etc.) associated with different user activities (e.g., computer interactions, watching content, exercise such as yoga, vacuuming, etc.) occurring during different time periods (e.g., yoga occurring between about 200-400 seconds, etc.). Likewise, audio waveform data 304 represents different audio patterns/features that include extracted audio characteristics (e.g., volume, frequency bands, etc.) associated with each of the different user activities (e.g., a large audio wave pattern occurring during vacuuming).

In some implementations, gating result data 308 represents whether a context change has been detected. For example, 1 indicates that a context change has been detected and triggers image capture. Likewise, 0 indicates no context change resulting in no image capture. Therefore, when activity segmentation data 302 indicates a context change (at around 200 seconds) associated with a user transitioning from yoga to vacuuming, gating result data 308 indicates the transition resulting in image capture.

Accordingly, time-series dataset 300 represents captures at key transitions when transitioning activities to avoid unnecessary captures during continuous actions. Likewise, IMU and audio fusion enhances accuracy to ensure that both movement and audio changes are both considered to detect a context change.

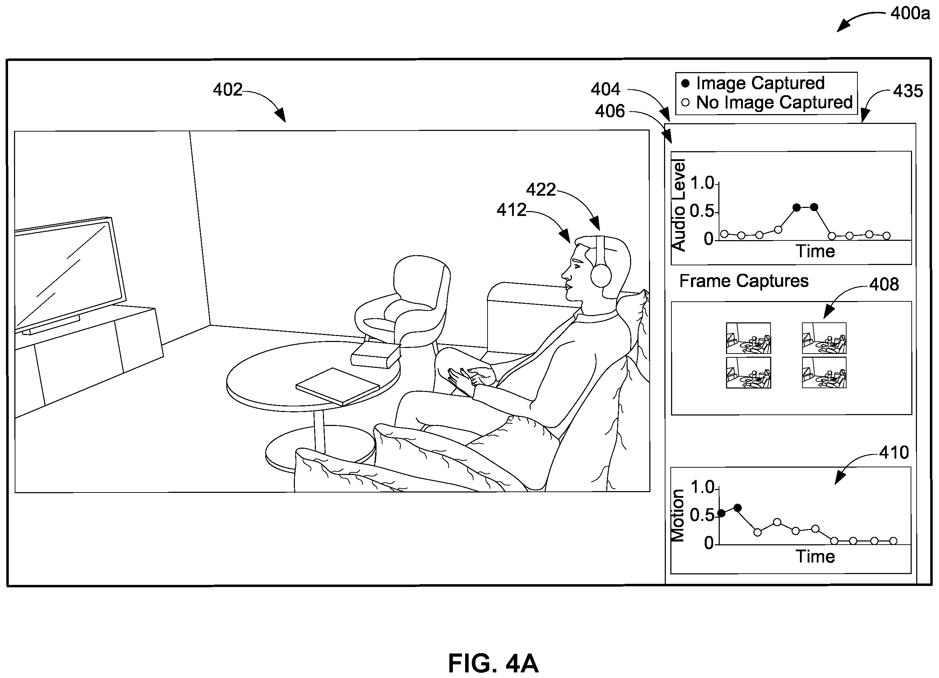

**Figures 4A-4C** illustrate views 400a-400c of a process implemented by the sensor gating framework 200 of Figure 2 for detecting transitions in user activity of a user 412 wearing a wearable device 422 in a physical environment 402 by monitoring low-power sensor data (e.g., IMU and audio data) and triggering or limiting the capture of images at select times, in accordance with some implementations. The wearable device 422 may be any type of wearable device such as, for example, a head mounted device (HMD), an audio headset (as illustrated in Figures 4A-4C), a smart watch, etc. The process illustrated in Figures 4A-4C may be implemented using any combination of devices such as wearable device 422, a mobile device 404 comprising image, IMU, and audio sensors, additional devices that include image, IMU, and audio sensors within physical environment 402, etc. For example, views 400a-400c illustrate a user 412 wearing a headset (i.e., wearable device 422) streaming audio. Likewise, user 412 may be using a mobile device 404 that includes IMU and audio sensors. The mobile device 404 may additionally include an IMU gating stack 410, an audio gating stack 406, captured images 408, and an interface 435. Interface 435 is configured to present IMU gating predictions of IMU gating stack 410, audio gating predictions of audio gating stack 406, and captured images 408.

In Figure 4A, at a first instant in time corresponding to view 400a, user 412 is determined to be sitting within a physical environment 402 while streaming audio via a headset (wearable device 422). Likewise, mobile device 404 is configured to run the gating models (IMU gating stack 410 and audio gating stack 406), process resulting data, and capture images when a context change is detected. In some implementations, IMU gating stack 410 and audio gating stack 406 may be periodically enabled (e.g., every 2 seconds) to detect context changes such that if a significant activity transition is detected, a gating signal is configured to trigger image capture. For example, view 400a illustrates that audio gating stack 406 has detected 2 significant context changes (e.g., a new sound in physical environment 402) resulting in 2 image captures. Likewise, view 400a illustrates that IMU gating stack 410 has detected 2 significant context changes (e.g., transitioning from sitting to walking) resulting in 2 image captures. Accordingly, captured images 408 in figure 4A includes 4 captured images as fusion logic for image capture may be configured to combine IMU and audio gating predictions and use a decision threshold to determine when and how many images to capture. Therefore, captured images 408 correspond to moments when gating signals fire. For example, if user 412 begins to play guitar, the process is configured to recognize this context and adapt its behavior accordingly such that instead of constantly capturing images, the process gates image capture based on context changes detected from IMU signals (e.g., hand movement patterns changing to guitar-playing motion) and audio signals (e.g., detecting distinct guitar strumming sounds). Accordingly, when a significant context shift is detected, an image(s) is selectively captured to validate the new activity.

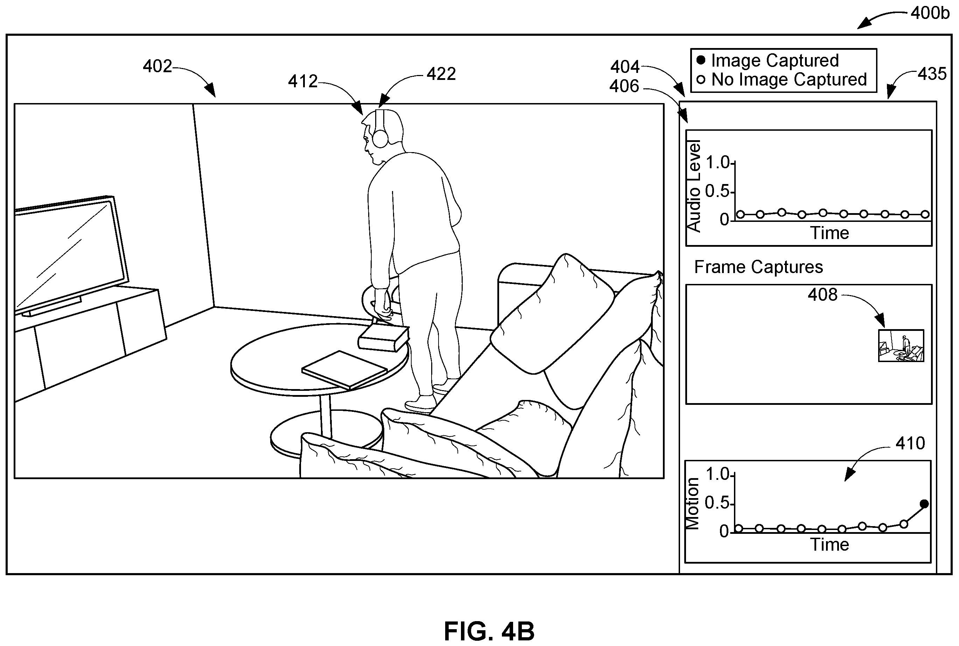

In Figure 4B at a second instant in time corresponding to view 400b, user 412 is determined to have transitioned from the sitting activity (of Figure 4A) to a standing activity as illustrated in Figure 4B. Accordingly, when user 412 stood up, the IMU gating stack 410 detected a significant context or movement transition thereby triggering a gating signal to initiate camera capture resulting in captured image 408 (e.g., 1 image in Figure 4B) to confirm the transition. For example, view 400b illustrates that audio gating stack 406 has not detected any significant context changes (e.g., no new sounds in physical environment 402). Likewise, view 400b illustrates that IMU gating stack 410 has detected 1 significant context change (e.g., transitioning from sitting to standing) resulting in image captures. Accordingly, captured images 408 includes multiple captured images as fusion logic for image capturing may be configured to use IMU gating predictions and to determine when and how many images to capture. Therefore, captured images 408 correspond to moments when gating signals fire.

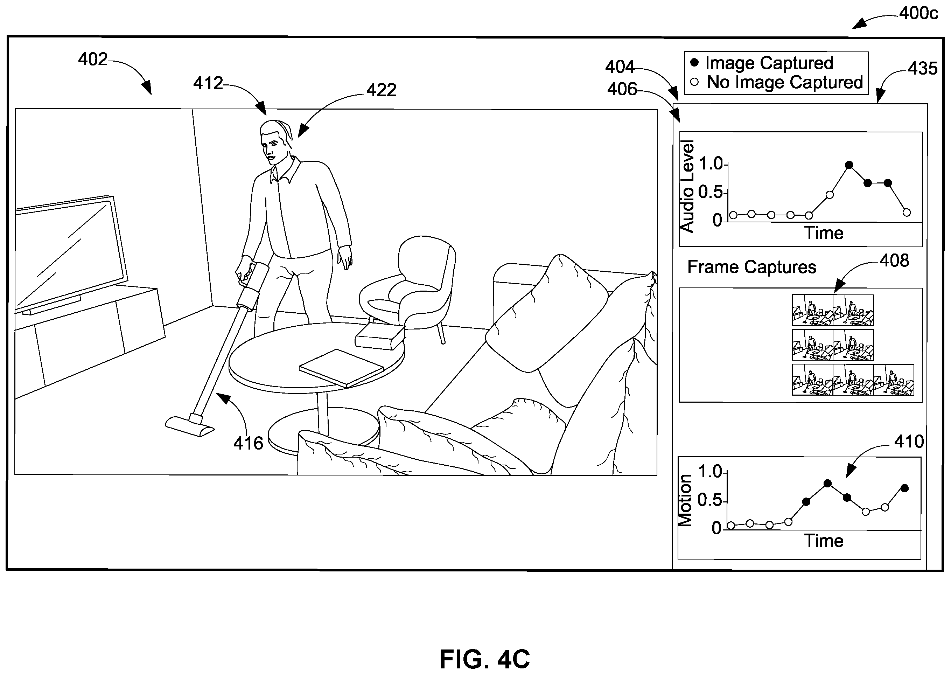

In Figure 4C at a third instant in time corresponding to view 400c, user 412 is determined to have transitioned from the standing activity (of Figure 4B) to a vacuuming activity as illustrated in Figure 4C. Accordingly, when user 412 started vacuuming, the IMU gating stack 410 and the audio gating stack 406 each detected multiple significant context or movement transitions thereby triggering gating signals to initiate camera capture resulting in captured images 408 (e.g., 7 images in figure 4C) to confirm the transition. For example, view 400c illustrates that audio gating stack 406 has detected multiple significant context changes (e.g., vacuum motor sounds in physical environment 402). Likewise, view 400c illustrates that IMU gating stack 410 has detected multiple significant context changes (e.g., transitioning from standing to vacuuming) resulting in image captures. Accordingly, captured images 408 includes multiple images as fusion logic for image capture may be configured to use IMU gating predictions and audio gating predictions to determine when and how many images to capture. Therefore, captured images 408 correspond to moments when gating signals fire.

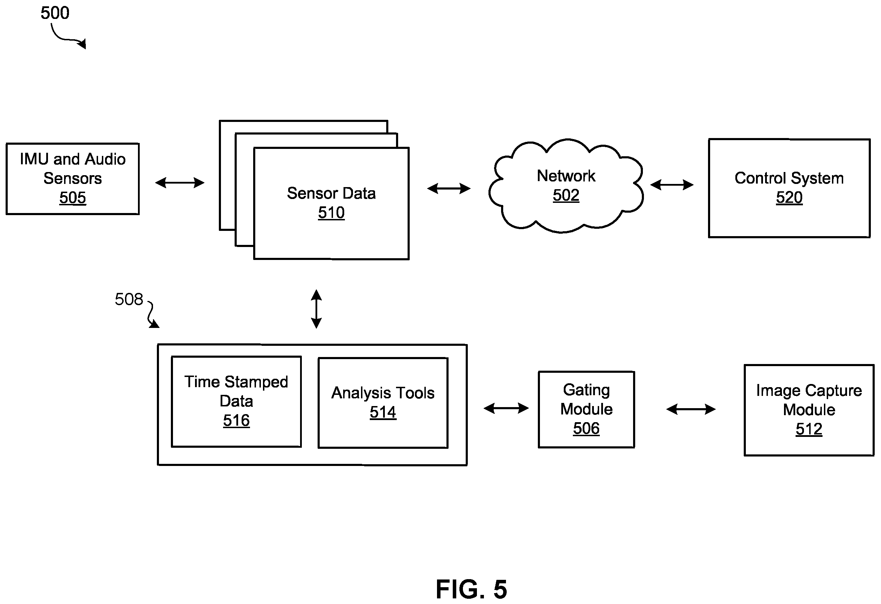

**Figure 5** illustrates a system 500 that leverages low power sensor data 510 obtained from IMU and audio sensors 405 to track and predict user activities over extended periods of time without continuously capturing image data, in accordance with some implementations. The system 500 includes IMU and audio sensors 505 (e.g., of and/or external to wearable device 422 and/or mobile device 404 of Figures 4A-4C) such as, *inter alia,* accelerometers, gyroscopes, microphones, etc. Likewise, system 500 includes tools/software 508, a gating module 506 controlling an image capture module 512 and a control system 520 that, in some implementations, communicates over a data communication network 502, e.g., a local area network (LAN), a wide area network (WAN), the Internet, a mobile network, or a combination thereof. Tools/software 508 comprise analysis tools 514 for analyzing time stamped data 516.

System 500 may be configured to enable low-power sensor data 510 collection using IMU and audio sensors 505 to monitor user movement and sound. Low-power sensor data 510 collection may enable continuous monitoring without significant battery drain. Subsequently, the sensor data 510 may be synchronized using timestamps and resulting timestamped data 516 may be periodically processed via analysis tools 514 to capture features over short time windows (e.g., every 2 seconds). This processed data may be aggregated (via analysis tools 514) over several time periods (e.g., 5 periods, corresponding to 10 seconds) to create a more comprehensive picture of the user's activity by detecting subtle changes in behavior.

In some implementations, two levels of information may be analyzed via analysis tools: (a) direct output from a feature extractor; and (b) higher-level semantic output. During analysis of direct output from a feature extractor, raw sensor data such as sensor data 510 may be analyzed to provide low-level insights about a user's immediate activity such as movement patterns or environmental sounds. Likewise, during analysis of higher-level semantic output, associated data may be analyzed to derive a higher-level understanding of the user's activity, such as detecting what specific action or activity the user is performing. This analysis may include a probability distribution for various activities (e.g., walking, sitting, talking, etc.) to provide insights into a most likely activity at a given moment. The two levels of information (raw features and semantic predictions) may be concatenated into a single information block for input to gating module 506 configured to determine when to capture images and/or trigger higher computational processes based on a determined change in context. For example, if system 500 detects a change from sitting to standing, an image capture may be triggered (via image capture module 512) to confirm the activity. Likewise, if system 500 detects no significant activity, no image capture is triggered. Accordingly, system 500 reduces constant image capture to save on computational resources and battery life.

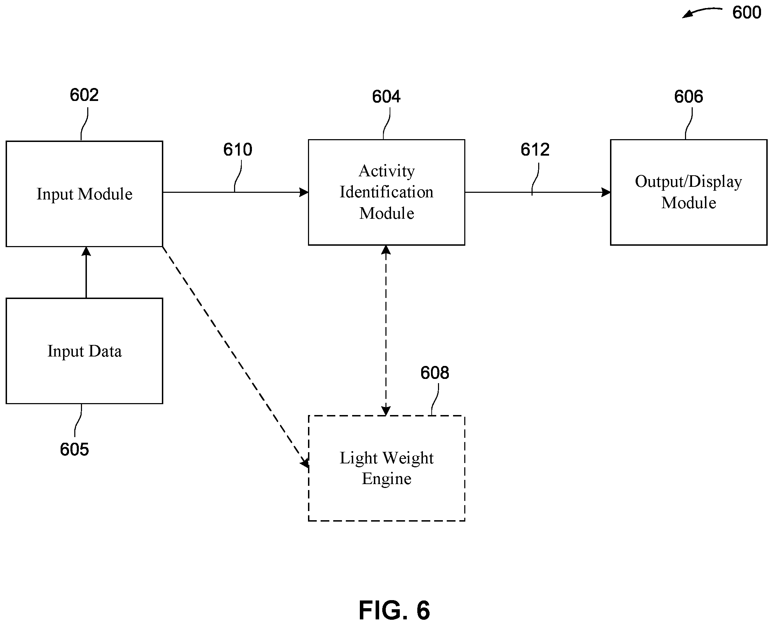

**Figure 6** is a block diagram of an example system illustrating a user device 600 configured to detect a change in user activity by monitoring low-power sensor data and triggering the capture of images at select times for processing to identify the user's new activity. The user device 600 (e.g., a wearable device) may include an input module 602, an activity identification module 604, an output/display module 606, and a light weight engine 608.

In some embodiments, input module 602 is configured to accept input data 605 (e.g., internal or external sensor data from IMU and audio sensors of and/or external to the user device 600) associated with user movements and audible sounds in a physical environment.

Input 610 from input module 602 can be fed to activity identification module 604. Activity identification module 604 can include one or more learning-based and/or non-learning-based models for perceiving, synthesizing, and inferring information for detecting a user activity change and applying rule-based image capture and analysis. Persons skilled in the art will appreciate that the activity identification module 604 can include any suitable number of rule-based process(es) to generate output 612 based on input 610.

In some embodiments, activity identification module 604 may be used to deterministically detect a user activity transition using an algorithmic rule-based approach to enable sensor fusion (IMU, accelerometer, gyroscope, etc.) to determine user movement patterns, detect contextual changes (e.g., phone picked up, headset movement, exercise), and store previous activity to compare with real-time data to detect transitions and apply rule-based image capture such that if an activity transition matches a defined rule, image capture may be triggered using selected camera(s) of, for example, a wearable device. Likewise, an algorithmic rule-based approach may be used to process a captured image using, for example, computer vision (CV) techniques to analyze the captured image and determine new user activity (e.g., sitting, standing, walking, interacting with an object, etc.).

In some embodiments, user device 600 may optionally elect to use a light weight engine 408 instead of activity identification module 604 to detect a user activity transition to determine user movement patterns, detect contextual changes and store previous activity to compare with real-time data to detect transitions and apply rule-based image capture such that if an activity transition matches a defined rule, image capture may be triggered using selected camera(s) of, for example, a wearable device. The light-weight engine 608 may be a non-learning network.

In some embodiments, output/display module 606 is configured to accept results from activity identification module 604 and/or a light weight engine 608.

For example, output from activity identification module 604 and/or a light weight engine 608 is provided to output/display module 606, where output/display module 606 provides a user experience on a wearable device.

Persons of ordinary skill in the art will appreciate that activity identification module 604 can include any suitable machine learning models that are well-known or widely available such as regression techniques, classification techniques, neural networks, and deep learning networks. For instance, activity identification module 604 can include neural networks such as Artificial Neural Network (ANN), Convolutional Neural Network (CNN), Recurrent Neural Network (RNN), Adversarial Network (GAN), Reinforcement Learning Model (RLM), Encoder/Decoder Networks, and/or Transformer-Based Models (e.g., Bidirectional Encoder Representations from Transformers (BERT), Generative Pre-trained Transformer (GPT), and/or a multimodal large language model (LLM)). Additionally or alternatively, persons of ordinary skill in the art will appreciate that activity identification module 604 can be any suitable non-learning processes such as rule-based systems, heuristics, decision trees, knowledge-based systems, statistical or stochastic systems, and expert systems.

In instances where activity identification module 604 is a machine-learning based model, the model may be trained to enable detect a change in user activity (context change) by monitoring low-power sensor data (e.g., IMU and audio data) and triggering the capture of images at select times (e.g., subsequent to a detected user activity change). Subsequently, the capture images may be processed (via computationally-heavier processes such as LLMs) to identify the user's new activity.

In some embodiments, activity identification module 604 can be trained with both synthetic and non-synthetic data.

In some embodiments, activity identification module 604 can be deployed as one or more generative models, where content is automatically generated by one or more computers in response to a request to generate the content. The automatically-generated content is optionally generated on-device (e.g., generated at least in part by a computer system at which a request to generate the content is received) and/or generated off-device (e.g., generated at least in part by one or more nearby computers that are available via a local network or one or more computers that are available via the internet). This automatically-generated content optionally may include visual content (e.g., images, graphics, and/or video) and/or text content.

In some embodiments, novel automatically-generated content is referred to as generative content (e.g., generative images, generative graphics, generative video, and/or generative text). Generative content is typically generated based on a prompt input 610 to the activity identification module 604. Input module 602 optionally includes one or more pre-processing steps to adjust the input before it is used by an artificial intelligence (AI) model to generate an output image (e.g., adjustment to a user-provided prompt, creation of a system-generated prompt, and/or AI model selection). Display module 606 optionally includes one or more post-processing steps to adjust the output 612 by the activity identification module 604 (e.g., passing the AI model output to a different AI model, upscaling, downscaling, cropping, formatting, and/or adding or removing metadata) before the output 612 of the activity identification module 604 used for other purposes such as being provided to a different software process for further processing or being presented (e.g., visually) to a user.

A prompt input 610 for generating generative content can include one or more of: one or more words (e.g., a natural language prompt that is written or spoken), one or more images, one or more drawings, and/or one or more videos. Generative pre-trained transformer models are a type of LLM that can be effective at generating novel generative content based on a prompt input 610. In some embodiments, the activity identification module 604 uses a prompt input 610 that includes text to generate either different generative text, generative audio content, and/or generative visual content. In other embodiments, the activity identification module 604 uses a prompt input 610 that includes visual content and/or an audio content to generate generative text (e.g., a transcription of audio and/or a description of the visual content). In yet other embodiments, the activity identification module 604 uses a prompt input 610 that includes multiple types of content (e.g., text, images, audio, video, and/or other sensor data) to generate generative content. A prompt input 610 sometimes also includes values for one or more parameters indicating an importance of various parts of the prompt. Some prompt inputs 610 include a structured set of instructions that can be provided to the activity identification module 604 that include phrasing, a specified style, relevant context (e.g., starting point content and/or one or more examples), and/or a role for the activity identification module 604.

Generative content is generally based on the prompt but is not deterministically selected from pre-generated content and is, instead, generated using the prompt as a starting point. In some embodiments, pre-existing content (e.g., audio, text, and/or visual content) is used as part of the prompt for creating generative content (e.g., the pre-existing content is used as a starting point for creating the generative content). For example, a prompt input 610 could request that visual content be modified to include or exclude content specified by a prompt (e.g., removing an identified feature in the visual content, adding a feature to the visual content that is described in a prompt, changing a visual style of the visual content, and/or creating additional visual elements outside of a spatial or temporal boundary of the visual content that are based on the visual content). In some embodiments, a random or pseudo-random seed is used as part of the prompt input 610 for creating generative content (e.g., the random or pseudo-random seed content is used as a starting point for creating the generative content). For example, when generating an image from a diffusion model, a random noise pattern is iteratively denoised based on the prompt input 610 to generate an image. While specific types of activity identification module 604 have been described herein, it should be understood that a variety of a different activity identification module could be used to generate generative content based on a prompt.

In instances where activity identification module 604 is a non-learning system, non-learning-based activity identification module 604 can use a pre-defined set of rules or a pre-defined structure to make decisions based on the inputs that the process sees. For example, the activity identification module 604 can be used to guide a deterministic approach to detect a change in user activity by monitoring low-power sensor data (e.g., IMU and audio data) and trigger capture of images at select times for processing (via computationally-heavier processes such as LLMs) to identify a user's new activity. Likewise, audio and IMU data may be synchronized using time stamps and the time-stamped data may be considered in blocks of time and may be fed into a data extractor (e.g., extracting a feature every 2 seconds) and these extracted features may be aggregated for a block of time (e.g., the last 5 periods corresponding to 10 seconds). Likewise, two levels of information may be analyzed. For example, direct output from a feature extractor and output from another process at a higher semantic level to provide an activity prediction. These two inputs may be concatenated and fed into a gating process to limit image capture.

Some embodiments described herein can include use of learning and/or non-learning-based process(es). The use can include collecting, pre-processing, encoding, labeling, organizing, analyzing, recommending and/or generating data. Entities that collect, share, and/or otherwise utilize user data should provide transparency and/or obtain user consent when collecting such data. The present disclosure recognizes that the use of the data in rule-based or machine learning (ML) based process(es) can be used to benefit users. For example, the data can be used to train models that can be deployed to improve performance, accuracy, and/or functionality of applications and/or services. Accordingly, the use of the data enables the rule-based process(es) or ML to adapt and/or optimize operations to provide more personalized, efficient, and/or enhanced user experiences. Such adaptation and/or optimization can include tailoring content, recommendations, and/or interactions to individual users, as well as streamlining processes, and/or enabling more intuitive interfaces. Further beneficial uses of the data in the rule-based process(es) can be used to are also contemplated by the present disclosure.

The present disclosure contemplates that, in some embodiments, data used by the rule-based process(es) can be used to include publicly available data. To protect user privacy, data may be anonymized, aggregated, and/or otherwise processed to remove or to the degree possible limit any individual identification. As discussed herein, entities that collect, share, and/or otherwise utilize such data should obtain user consent prior to and/or provide transparency when collecting such data. Furthermore, the present disclosure contemplates that the entities responsible for the use of data, including, but not limited to data used in association with the rule-based process(es), should attempt to comply with well-established privacy policies and/or privacy practices.

For example, such entities may implement and consistently follow policies and practices recognized as meeting or exceeding industry standards and regulatory requirements for developing and/or training ML-based process(es). In doing so, attempts should be made to ensure all intellectual property rights and privacy considerations are maintained. Training should include practices safeguarding training data, such as personal information, through sufficient protections against misuse or exploitation. Such policies and practices should cover all stages of the process(es) development, training, and use, including data collection, data preparation, model training, model evaluation, model deployment, and ongoing monitoring and maintenance. Transparency and accountability should be maintained throughout. Such policies should be easily accessible by users and should be updated as the collection and/or use of data changes. User data should be collected for legitimate and reasonable uses of the entity and not shared or sold outside of those legitimate uses. Further, such collection and sharing should occur through transparency with users and/or after receiving the informed consent of the users. Additionally, such entities should consider taking any needed steps for safeguarding and securing access to such data and ensuring that others with access to the data adhere to their privacy policies and procedures. Further, such entities should subject themselves to evaluation by third parties to certify, as appropriate for transparency purposes, their adherence to widely accepted privacy policies and practices. In addition, policies and/or practices should be adapted to the particular type of data being collected and/or accessed and tailored to a specific use case and applicable laws and standards, including jurisdiction-specific considerations.

In some embodiments, ML-based process(es) may utilize models that may be trained (e.g., supervised learning or unsupervised learning) using various training data, including data collected using a user device. Such use of user-collected data may be limited to operations on the user device. For example, the training of the model can be done locally on the user device so no part of the data is sent to another device. In other implementations, the training of the model can be performed using one or more other devices (e.g., server(s)) in addition to the user device but done in a privacy preserving manner, e.g., via multi-party computation as may be done cryptographically by secret sharing data or other means so that the user data is not leaked to the other devices.

In some embodiments, the trained model can be centrally stored on the user device or stored on multiple devices, e.g., as in federated learning. Such decentralized storage can similarly be done in a privacy preserving manner, e.g., via cryptographic operations where each piece of data is broken into shards such that no device alone (i.e., only collectively with another device(s)) or only the user device can reassemble or use the data. In this manner, a pattern of behavior of the user or the device may not be leaked, while taking advantage of increased computational resources of the other devices to train and execute the ML model. Accordingly, user-collected data can be protected. In some implementations, data from multiple devices can be combined in a privacy-preserving manner to train an ML model.

In some embodiments, the present disclosure contemplates that data used for activity identification module 604 may be kept strictly separated from platforms where the activity identification module 604 are deployed and/or used to interact with users and/or process data. In such embodiments, data used for offline training of the activity identification module 604 may be maintained in secured datastores with restricted access and/or not be retained beyond the duration necessary for training purposes. In some embodiments, the activity identification module 604 may utilize a local memory cache to store data temporarily during a user session. The local memory cache may be used to improve performance of the rule-based process(es). However, to protect user privacy, data stored in the local memory cache may be erased after the user session is completed. Any temporary caches of data used for online learning or inference may be promptly erased after processing. All data collection, transfer, and/or storage should use industry-standard encryption and/or secure communication.

In some embodiments, as noted above, techniques such as federated learning, differential privacy, secure hardware components, homomorphic encryption, and/or multi-party computation among other techniques may be utilized to further protect personal information data during ML training and/or use of rule-based process(es). The activity identification module 604 should be monitored for changes in underlying data distribution such as concept drift or data skew that can degrade performance of the activity identification module 604 over time.

In some embodiments, the activity identification module 604 are trained using a combination of offline and online training. Offline training can use curated datasets to establish baseline model performance, while online training can allow the activity identification module 604 to continually adapt and/or improve. The present disclosure recognizes the importance of maintaining strict data governance practices throughout this process to ensure user privacy is protected.

In some embodiments, the activity identification module 604 processes may be designed with safeguards to maintain adherence to originally intended purposes, even as the activity identification module 604 adapt based on new data. Any significant changes in data collection and/or applications of activity identification module 604 use may (and in some cases should) be transparently communicated to affected stakeholders and/or include obtaining user consent with respect to changes in how user data is collected and/or utilized.

Despite the foregoing, the present disclosure also contemplates embodiments in which users selectively restrict and/or block the use of and/or access to data. That is, the present disclosure contemplates that hardware and/or software elements can be provided to prevent or block access to data. For example, in the case of some services, the present technology should be configured to allow users to select to "opt in" or "opt out" of participation in the collection of data during registration for services or anytime thereafter. In another example, the present technology should be configured to allow users to select not to provide certain data for training the activity identification module 604 and/or for use as input during the inference stage of such systems. In yet another example, the present technology should be configured to allow users to be able to select to limit the length of time data is maintained or entirely prohibit the use of their data for use by the rule-based process(es). In addition to providing "opt in" and "opt out" options, the present disclosure contemplates providing notifications relating to the access or use of personal information. For instance, a user can be notified when their data is being input into the activity identification module 604 for training or inference purposes, and/or reminded when the activity identification module 604 generate outputs or make decisions based on their data.

The present disclosure recognizes activity identification module 604 should incorporate explicit restrictions and/or oversight to mitigate against risks that may be present even when such systems having been designed, developed, and/or operated according to industry best practices and standards. For example, outputs may be produced that could be considered erroneous, harmful, offensive, and/or biased; such outputs may not necessarily reflect the opinions or positions of the entities developing or deploying these systems. Furthermore, in some cases, references to or failures to cite third-party products and/or services in the outputs should not be construed as endorsements or affiliations by the entities providing the rule-based process(es). Generated content can be filtered for potentially inappropriate or dangerous material prior to being presented to users, while human oversight and/or ability to override or correct erroneous or undesirable outputs can be maintained as a failsafe.

The present disclosure further contemplates that users of the activity identification module 604 should refrain from using the services in any manner that infringes upon, misappropriates, or violates the rights of any party. Furthermore, the activity identification module 604 should not be used for any unlawful or illegal activity, nor to develop any application or use case that would commit or facilitate the commission of a crime, or other tortious, unlawful, or illegal act including misinformation, disinformation, misrepresentations (e.g., deepfakes), deception, impersonation, and propaganda. The activity identification module 604 should not violate, misappropriate, or infringe any copyrights, trademarks, rights of privacy and publicity, trade secrets, patents, or other proprietary or legal rights of any party, and appropriately attribute content as required. Further, the activity identification module 604 should not interfere with any security, digital signing, digital rights management, content protection, verification, or authentication mechanisms. The activity identification module 604 should not misrepresent machine-generated outputs as being human-generated.

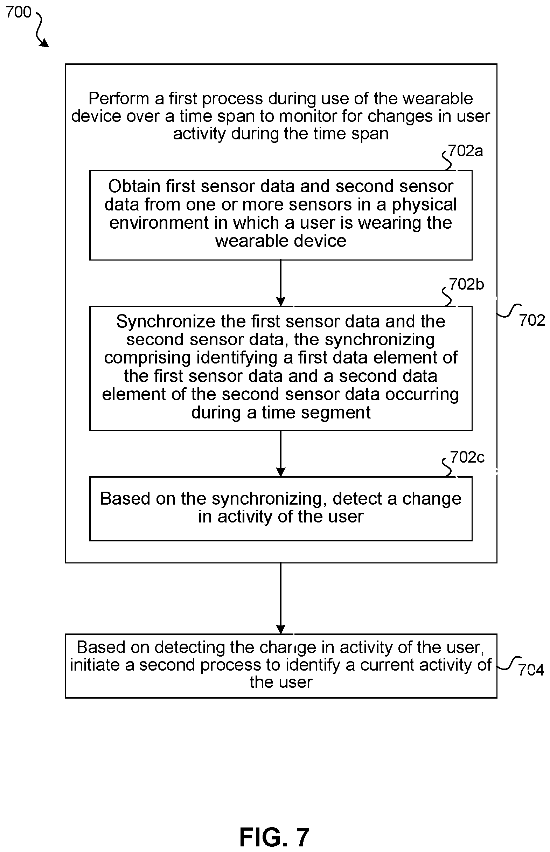

**Figure 7** is a flowchart representation of an exemplary method 700 that detects a change in user activity by monitoring low-power sensor data and triggers the capture of images at select times for processing to identify the user's new activity, in accordance with some implementations. In some implementations, the method 700 is performed by a wearable device such as wearable devices 105 and 110 of Figure 1 and/or wearable device 422 of Figures 4A-4C. In some implementations, the wearable device has a screen for displaying images and/or a screen for viewing stereoscopic images such as a head-mounted display (an HMD such as e.g., device 105 of Figure 1), an OST-AR device, etc. In some implementations, the wearable device is an audio headset. In some implementations, the method 700 is performed by processing logic, including hardware, firmware, software, or a combination thereof. In some implementations, the method 700 is performed by a processor executing code stored in a non-transitory computer-readable medium (e.g., a memory). Each of the blocks in the method 700 may be enabled and executed in any order.

At block 702, the method 700 performs a first process (including blocks 702a, 702b and 702c) during use of a wearable device over a time span to monitor for changes in user activity during the time span. For example, detecting transitions in user activity of a user 412 wearing a wearable device (e.g., headset) in a physical environment 402 by monitoring low-power sensor data such as IMU and audio data as described with respect to Figures 4A-4C.

At block 702a, the method obtains first sensor data and second sensor data from sensors in a physical environment in which a user is wearing the wearable device.

At block 702b, the method synchronizes the first sensor data and the second sensor data. In some implementations, synchronizing the first sensor data and the second sensor data may include identifying a first data element of the first sensor data and a second data element of the second sensor data occurring during a time segment. In some implementations, identifying the first data element may include identifying an occurrence of motion of the user and identifying the second data element may include identifying an occurrence of an audible sound. In some implementations, synchronizing the first sensor data and the second sensor data may include periodically feeding a first signal comprising the first sensor data and a second signal comprising the second sensor data into a data extractor to extract features for each of a plurality of time segments. In some implementations, the extracted features may be aggregated for a block of time that includes multiple time segments of the plurality of time segments and detecting the change in activity of the user occurring during the block of time based on analyzing the aggregated extracted features for the block of time. For example, as described with respect to Figure 5, sensor data 510 may be synchronized using timestamps and the timestamped data 516 may be periodically processed via analysis tools 514 to capture features over short time windows (e.g., every 2 seconds). This processed data may be aggregated (via analysis tools 514) over several time periods (e.g., 5 periods, corresponding to 10 seconds) to create a more comprehensive picture of the user's activity by detecting subtle changes in behavior as further described with respect to Figure 5.

At block 702c, the method detects a change in activity of the user based on synchronizing the first sensor data and the second sensor data. In some implementations, detecting the change in activity of the user may include, *inter alia,* detecting a change in a motion type of the user, detecting a change in a pattern of motion of the user, detecting a change in an audible sound associated with the user, etc.

In some implementations, the first sensor data and the second sensor data each comprise low power sensor data such as, for example, inertial measurement unit (IMU) data, audio data, etc.

At block 704, the method 700 initiates a second process to identify a current activity of the user based on detecting the change in activity of the user. For example, IMU and audio gating models of a gating module 202 may be configured to trigger an image capture sequence for analysis to identify a current activity or activity change as described with respect to Figure 2.

In some implementations, initiating the second process may include obtaining at least one image from an image sensor and initiating a computationally heavy process (e.g., an LLM) with respect to identifying the current activity of the user based on the at least one image. In some implementations, power requirements of utilizing the first process for a given length of time are less than power requirements of utilizing the second process for the given length of time. The second process may only be initiated during circumstances in which a change in the user activity is identified. In some implementations, the first process may be used to gate utilization of the second process to conserve resource utilization on the wearable device.

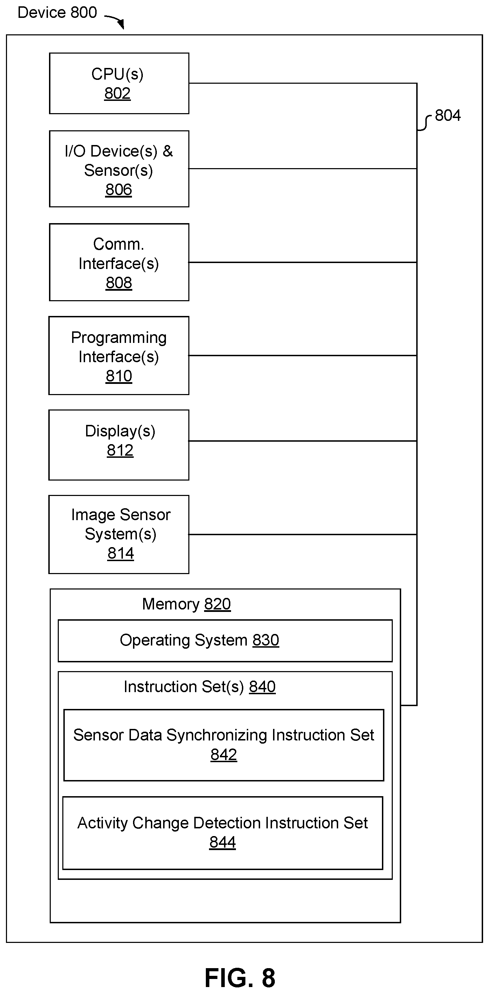

**Figure 8** is a block diagram of an example device 800. Device 800 illustrates an exemplary device configuration for electronic devices 105 and 110 of Figure 1. While certain specific features are illustrated, those skilled in the art will appreciate from the present disclosure that various other features have not been illustrated for the sake of brevity, and so as not to obscure more pertinent aspects of the implementations disclosed herein. To that end, as a non-limiting example, in some implementations the device 800 includes one or more processing units 802 (e.g., microprocessors, ASICs, FPGAs, GPUs, CPUs, processing cores, and/or the like), one or more input/output (I/O) devices and sensors 806, one or more communication interfaces 808 (e.g., USB, FIREWIRE, THUNDERBOLT, IEEE 802.3x, IEEE 802.1 1x, IEEE 802.16x, GSM, CDMA, TDMA, GPS, IR, BLUETOOTH, ZIGBEE, SPI, I2C, and/or the like type interface), one or more programming (e.g., I/O) interfaces 810, one or more displays 812, one or more interior and/or exterior facing image sensor systems 814, a memory 820, and one or more communication buses 804 for interconnecting these and various other components.

In some implementations, the one or more communication buses 804 include circuitry that interconnects and controls communications between system components. In some implementations, the one or more I/O devices and sensors 806 include at least one of an inertial measurement unit (IMU), an accelerometer, a magnetometer, a gyroscope, a thermometer, one or more physiological sensors (e.g., blood pressure monitor, heart rate monitor, blood oxygen sensor, blood glucose sensor, etc.), one or more microphones, one or more speakers, a haptics engine, one or more depth sensors (e.g., a structured light, a time-of-flight, or the like), and/or the like.

In some implementations, the one or more displays 812 are configured to present a view of a physical environment or a graphical environment to the user. In some implementations, the one or more displays 812 are configured to present content (determined based on a determined user/object location of the user within the physical environment) to the user. In some implementations, the one or more displays 812 correspond to holographic, digital light processing (DLP), liquid-crystal display (LCD), liquid-crystal on silicon (LCoS), organic light-emitting field-effect transitory (OLET), organic light-emitting diode (OLED), surface-conduction electron-emitter display (SED), field-emission display (FED), quantum-dot light-emitting diode (QD-LED), micro-electromechanical system (MEMS), and/or the like display types. In some implementations, the one or more displays 812 correspond to diffractive, reflective, polarized, holographic, etc. waveguide displays. In one example, the device 800 includes a single display. In another example, the device 800 includes a display for each eye of the user.

In some implementations, the one or more image sensor systems 814 are configured to obtain image data that corresponds to at least a portion of the physical environment 105. For example, the one or more image sensor systems 814 include one or more RGB cameras (e.g., with a complimentary metal-oxide-semiconductor (CMOS) image sensor or a charge-coupled device (CCD) image sensor), monochrome cameras, IR cameras, depth cameras, event-based cameras, and/or the like. In various implementations, the one or more image sensor systems 814 further include illumination sources that emit light, such as a flash. In various implementations, the one or more image sensor systems 814 further include an on-camera image signal processor (ISP) configured to execute a plurality of processing operations on the image data.

In some implementations, sensor data may be obtained by device(s) (e.g., devices 105 and 110 of Figure 1) during a scan of a room of a physical environment. The sensor data may include a 3D point cloud and a sequence of 2D images corresponding to captured views of the room during the scan of the room. In some implementations, the sensor data includes image data (e.g., from an RGB camera), depth data (e.g., a depth image from a depth camera), ambient light sensor data (e.g., from an ambient light sensor), and/or motion data from one or more motion sensors (e.g., accelerometers, gyroscopes, IMU, etc.). In some implementations, the sensor data includes visual inertial odometry (VIO) data determined based on image data. The 3D point cloud may provide semantic information about one or more elements of the room. The 3D point cloud may provide information about the positions and appearance of surface portions within the physical environment. In some implementations, the 3D point cloud is obtained over time, e.g., during a scan of the room, and the 3D point cloud may be updated, and updated versions of the 3D point cloud obtained over time. For example, a 3D representation may be obtained (and analyzed/processed) as it is updated/adjusted over time (e.g., as the user scans a room).

In some implementations, sensor data may be positioning information, some implementations include a VIO to determine equivalent odometry information using sequential camera images (e.g., light intensity image data) and motion data (e.g., acquired from the IMU/motion sensor) to estimate the distance traveled. Alternatively, some implementations of the present disclosure may include a simultaneous localization and mapping (SLAM) system (e.g., position sensors). The SLAM system may include a multidimensional (e.g., 3D) laser scanning and range-measuring system that is GPS independent and that provides real-time simultaneous location and mapping. The SLAM system may generate and manage data for a very accurate point cloud that results from reflections of laser scanning from objects in an environment. Movements of any of the points in the point cloud are accurately tracked over time, so that the SLAM system can maintain precise understanding of its location and orientation as it travels through an environment, using the points in the point cloud as reference points for the location.

In some implementations, the device 800 includes an eye tracking system for detecting eye position and eye movements (e.g., eye gaze detection). For example, an eye tracking system may include one or more infrared (IR) light-emitting diodes (LEDs), an eye tracking camera (e.g., near-IR (NIR) camera), and an illumination source (e.g., an NIR light source) that emits light (e.g., NIR light) towards the eyes of the user. Moreover, the illumination source of the device 800 may emit NIR light to illuminate the eyes of the user and the NIR camera may capture images of the eyes of the user. In some implementations, images captured by the eye tracking system may be analyzed to detect position and movements of the eyes of the user, or to detect other information about the eyes such as pupil dilation or pupil diameter. Moreover, the point of gaze estimated from the eye tracking images may enable gaze-based interaction with content shown on the near-eye display of the device 800.

The memory 820 includes high-speed random-access memory, such as DRAM, SRAM, DDR RAM, or other random-access solid-state memory devices. In some implementations, the memory 820 includes non-volatile memory, such as one or more magnetic disk storage devices, optical disk storage devices, flash memory devices, or other non-volatile solid-state storage devices. The memory 820 optionally includes one or more storage devices remotely located from the one or more processing units 802. The memory 820 includes a non-transitory computer readable storage medium.

In some implementations, the memory 820 or the non-transitory computer readable storage medium of the memory 820 stores an optional operating system 830 and one or more instruction set(s) 840. The operating system 830 includes procedures for handling various basic system services and for performing hardware dependent tasks. In some implementations, the instruction set(s) 840 include executable software defined by binary information stored in the form of electrical charge. In some implementations, the instruction set(s) 840 are software that is executable by the one or more processing units 802 to carry out one or more of the techniques described herein.

The instruction set(s) 840 includes a sensor data synchronizing instruction set 842 and an activity change detection instruction set 844. The instruction set(s) 840 may be embodied as a single software executable or multiple software executables.

The sensor data synchronizing instruction set 842 is configured with instructions executable by a processor to synchronize different types of sensor data by identifying data elements of the sensor data occurring during a time segment.

The activity change detection instruction set 844 is configured with instructions executable by a processor to detecting a change in activity of a user to identify a current activity of the user.

Although the instruction set(s) 840 are shown as residing on a single device, it should be understood that in other implementations, any combination of the elements may be located in separate computing devices. Moreover, Figure 8 is intended more as functional description of the various features which are present in a particular implementation as opposed to a structural schematic of the implementations described herein. As recognized by those of ordinary skill in the art, items shown separately could be combined and some items could be separated. The actual number of instructions sets and how features are allocated among them may vary from one implementation to another and may depend in part on the particular combination of hardware, software, and/or firmware chosen for a particular implementation.

Those of ordinary skill in the art will appreciate that well-known systems, methods, components, devices, and circuits have not been described in exhaustive detail so as not to obscure more pertinent aspects of the example implementations described herein. Moreover, other effective aspects and/or variants do not include all of the specific details described herein. Thus, several details are described in order to provide a thorough understanding of the example aspects as shown in the drawings. Moreover, the drawings merely show some example embodiments of the present disclosure and are therefore not to be considered limiting.

While this specification contains many specific implementation details, these should not be construed as limitations on the scope of any inventions or of what may be claimed, but rather as descriptions of features specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described. Other embodiments are within the scope of the following claims. In some cases, the actions recited in the claims can be performed in a different order and still achieve desirable results. In addition, the processes depicted in the accompanying Figures do not necessarily require the particular order shown, or sequential order, to achieve desirable results. In certain implementations, multitasking and parallel processing may be advantageous.

Embodiments of the subject matter and the operations described in this specification can be implemented in digital electronic circuitry, or in computer software, firmware, or hardware, including the structures disclosed in this specification and their structural equivalents, or in combinations of one or more of them. Embodiments of the subject matter described in this specification can be implemented as one or more computer programs, e.g., one or more modules of computer program instructions, encoded on computer storage medium for execution by, or to control the operation of, data processing apparatus. Alternatively, or additionally, the program instructions can be encoded on an artificially generated propagated signal, e.g., a machine-generated electrical, optical, or electromagnetic signal, that is generated to encode information for transmission to suitable receiver apparatus for execution by a data processing apparatus. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially generated propagated signal. The computer storage medium can also be, or be included in, one or more separate physical components or media (e.g., multiple CDs, disks, or other storage devices).

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures. Unless specifically stated otherwise, it is appreciated that throughout this specification discussions utilizing the terms such as "processing," "computing," "calculating," "determining," and "identifying" or the like refer to actions or processes of a computing device, such as one or more computers or a similar electronic computing device or devices, that manipulate or transform data represented as physical electronic or magnetic quantities within memories, registers, or other information storage devices, transmission devices, or display devices of the computing platform.

The system or systems discussed herein are not limited to any particular hardware architecture or configuration. A computing device can include any suitable arrangement of components that provides a result conditioned on one or more inputs. Suitable computing devices include multipurpose microprocessor-based computer systems accessing stored software that programs or configures the computing system from a general purpose computing apparatus to a specialized computing apparatus implementing one or more implementations of the present subject matter. Any suitable programming, scripting, or other type of language or combinations of languages may be used to implement the teachings contained herein in software to be used in programming or configuring a computing device.

Implementations of the methods disclosed herein may be performed in the operation of such computing devices. The order of the blocks presented in the examples above can be varied for example, blocks can be re-ordered, combined, and/or broken into sub-blocks. Certain blocks or processes can be performed in parallel. The operations described in this specification can be implemented as operations performed by a data processing apparatus on data stored on one or more computer-readable storage devices or received from other sources.

The use of "adapted to" or "configured to" herein is meant as open and inclusive language that does not foreclose devices adapted to or configured to perform additional tasks or steps. Additionally, the use of "based on" is meant to be open and inclusive, in that a process, step, calculation, or other action "based on" one or more recited conditions or values may, in practice, be based on additional conditions or value beyond those recited. Headings, lists, and numbering included herein are for ease of explanation only and are not meant to be limiting.

It will also be understood that, although the terms "first," "second," etc. may be used herein to describe various elements, these elements should not be limited by these terms. These terms are only used to distinguish one element from another. For example, a first node could be termed a second node, and, similarly, a second node could be termed a first node, which changing the meaning of the description, so long as all occurrences of the "first node" are renamed consistently and all occurrences of the "second node" are renamed consistently. The first node and the second node are both nodes, but they are not the same node.

The terminology used herein is for the purpose of describing particular implementations only and is not intended to be limiting of the claims. As used in the description of the implementations and the appended claims, the singular forms "a," "an," and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" may be construed to mean "when" or "upon" or "in response to determining" or "in accordance with a determination" or "in response to detecting," that a stated condition precedent is true, depending on the context. Similarly, the phrase "if it is determined [that a stated condition precedent is true]" or "if [a stated condition precedent is true]" or "when [a stated condition precedent is true]" may be construed to mean "upon determining" or "in response to determining" or "in accordance with a determination" or "upon detecting" or "in response to detecting" that the stated condition precedent is true, depending on the context.

Exemplary methods, systems, wearable devices, and non-transitory computer-readable storage media are set out in the following items.

### Item list 1:

1. A method comprising:
   at a wearable device having a processor:
   performing a first process during use of the wearable device over a time span to monitor for changes in user activity during the time span, the first process comprising:
      obtaining first sensor data and second sensor data from one or more sensors in a physical environment in which a user is wearing the wearable device;
      synchronizing the first sensor data and the second sensor data, the synchronizing comprising identifying a first data element of the first sensor data and a second data element of the second sensor data occurring during a time segment; and
      based on the synchronizing, detecting a change in activity of the user; and
   based on detecting the change in activity of the user, initiating a second process to identify a current activity of the user.
2. The method of item 1, wherein the synchronizing comprises periodically feeding a first signal comprising the first sensor data and a second signal comprising the second sensor data into a data extractor, wherein the data extractor extracts features for each of a plurality of time segments.
3. The method of item 2 further comprising:
   aggregating the extracted features for a block of time comprising multiple time segments of the plurality of time segments; and
   detecting the change in activity of the user occurring during the block of time based on analyzing the aggregated extracted features for the block of time.
4. The method of any of items 1-3, wherein the first sensor data and the second sensor data each comprise low power sensor data.
5. The method of any of items 1-4, wherein the first sensor data comprises inertial measurement unit (IMU) data and second sensor data comprises audio data.
6. The method of any of items 1-5, wherein identifying the first data element comprises identifying an occurrence of motion of the user, and wherein identifying the second data element comprises identifying an occurrence of an audible sound.
7. The method of any of items 1-6, wherein detecting the change in activity of the user comprises detecting a change in a motion type of the user.
8. The method of any of items 1-7, wherein detecting the change in activity of the user comprises detecting a change in a pattern of motion of the user.
9. The method of any of items 1-8, wherein detecting the change in activity of the user comprises detecting a change in an audible sound associated with the user.
10. The method of any of items 1-9, wherein initiating the second process comprises initiating obtaining at least one image from an image sensor.
11. The method of any of items 1-9, wherein initiating the second process comprises initiating computationally heavy process with respect to identifying the current activity of the user.
12. The method of item 11, wherein the computationally heavy process comprises a large language model-based process.
13. The method of any of items 1-12, wherein power requirements of utilizing the first process for a given length of time are less than power requirements of utilizing the second process for the given length of time.
14. The method of any of items 1-13, wherein the second process is only initiated in circumstances in which a change in the user activity is identified.
15. The method of any of item 1-4, wherein the first process is used to gate utilization of the second process to conserve resource utilization on the wearable device.
16. A wearable device comprising:
   a non-transitory computer-readable storage medium; and
   one or more processors coupled to the non-transitory computer-readable storage medium, wherein the non-transitory computer-readable storage medium comprises program instructions that, when executed on the one or more processors, cause the wearable device to perform operations comprising:
      performing a first process during use of the wearable device over a time span to monitor for changes in user activity during the time span, the first process comprising:
         obtaining first sensor data and second sensor data from one or more sensors in a physical environment in which a user is wearing the wearable device;
         synchronizing the first sensor data and the second sensor data, the synchronizing comprising identifying a first data element of the first sensor data and a second data element of the second sensor data occurring during a time segment; and
         based on the synchronizing, detecting a change in activity of the user; and
      based on detecting the change in activity of the user, initiating a second process to identify a current activity of the user.
17. The wearable device of item 16, wherein the synchronizing comprises periodically feeding a first signal comprising the first sensor data and a second signal comprising the second sensor data into a data extractor, wherein the data extractor extracts features for each of a plurality of time segments.
18. The wearable device of item 17, wherein the program instructions, when executed on the one or more processors, further cause the wearable device to perform operations comprising:
   aggregating the extracted features for a block of time comprising multiple time segments of the plurality of time segments; and
   detecting the change in activity of the user occurring during the block of time based on analyzing the aggregated extracted features for the block of time.
19. The wearable device of any of items 16-18, wherein the first sensor data and the second sensor data each comprise low power sensor data.
20. A non-transitory computer-readable storage medium, storing program instructions executable by one or more processors to perform operations comprising:
   at a wearable electronic device having a processor:
   performing a first process during use of the wearable device over a time span to monitor for changes in user activity during the time span, the first process comprising:
   obtaining first sensor data and second sensor data from one or more sensors in a physical environment in which a user is wearing the wearable device;
   synchronizing the first sensor data and the second sensor data, the synchronizing comprising identifying a first data element of the first sensor data and a second data element of the second sensor data occurring during a time segment; and
   based on the synchronizing, detecting a change in activity of the user; and
   based on detecting the change in activity of the user, initiating a second process to identify a current activity of the user.

### Item list 2:

1. A method comprising:
   at a wearable device having a processor:
   performing a first process during use of the wearable device over a time span to monitor for changes in user activity during the time span, the first process comprising:
      obtaining first sensor data and second sensor data from one or more sensors in a physical environment in which a user is wearing the wearable device;
      synchronizing the first sensor data and the second sensor data, the synchronizing comprising identifying a first data element of the first sensor data and a second data element of the second sensor data occurring during a time segment; and
      based on the synchronizing, detecting a change in activity of the user; and
   based on detecting the change in activity of the user, initiating a second process to identify a current activity of the user.
2. The method of item 1, wherein the synchronizing comprises periodically feeding a first signal comprising the first sensor data and a second signal comprising the second sensor data into a data extractor, wherein the data extractor extracts features for each of a plurality of time segments.
3. The method of item 2 further comprising:
   aggregating the extracted features for a block of time comprising multiple time segments of the plurality of time segments; and
   detecting the change in activity of the user occurring during the block of time based on analyzing the aggregated extracted features for the block of time.
4. The method item 1, wherein the first sensor data and the second sensor data each comprise low power sensor data.
5. The method of item 1, wherein the first sensor data comprises inertial measurement unit (IMU) data and second sensor data comprises audio data.
6. The method of item 1, wherein identifying the first data element comprises identifying an occurrence of motion of the user, and wherein identifying the second data element comprises identifying an occurrence of an audible sound.
7. The method of item 1, wherein detecting the change in activity of the user comprises detecting a change in a motion type of the user.
8. The method of item 1, wherein detecting the change in activity of the user comprises detecting a change in a pattern of motion of the user.
9. The method of item 1, wherein detecting the change in activity of the user comprises detecting a change in an audible sound associated with the user.
10. The method of item 1, wherein initiating the second process comprises initiating obtaining at least one image from an image sensor.
11. The method of item 1, wherein initiating the second process comprises initiating computationally heavy process with respect to identifying the current activity of the user.
12. The method of item 11, wherein the computationally heavy process comprises a large language model-based process.
13. The method of item 1, wherein power requirements of utilizing the first process for a given length of time are less than power requirements of utilizing the second process for the given length of time.
14. A system comprising:
   a processor;
   a computer readable medium storing instructions that when executed by the processor cause the processor to perform operations comprising any one of the methods of items 1-13.
15. A non-transitory computer-readable medium comprising instructions that when executed by a processor cause the processor to perform operations comprising any one of the methods of items 1-13.

The foregoing description, for purpose of explanation, has been described with reference to specific embodiments. However, the illustrative discussions above are not intended to be exhaustive or to limit the invention to the precise forms disclosed. Many modifications and variations are possible in view of the above teachings. The embodiments were chosen and described in order to best explain the principles of the invention and its practical applications, to thereby enable others skilled in the art to best utilize the invention and various embodiments with various modifications as are suited to the particular use contemplated.

## Claims

1. A method comprising:
at a wearable device having a processor:
performing a first process during use of the wearable device over a time span to monitor for changes in user activity during the time span, the first process comprising:
obtaining first sensor data and second sensor data from one or more sensors in a physical environment in which a user is wearing the wearable device;
synchronizing the first sensor data and the second sensor data, the synchronizing comprising identifying a first data element of the first sensor data and a second data element of the second sensor data occurring during a time segment; and
based on the synchronizing, detecting a change in activity of the user; and
based on detecting the change in activity of the user, initiating a second process to identify a current activity of the user.

2. The method of claim 1, wherein the synchronizing comprises periodically feeding a first signal comprising the first sensor data and a second signal comprising the second sensor data into a data extractor, wherein the data extractor extracts features for each of a plurality of time segments.

3. The method of claim 2 further comprising:
aggregating the extracted features for a block of time comprising multiple time segments of the plurality of time segments; and
detecting the change in activity of the user occurring during the block of time based on analyzing the aggregated extracted features for the block of time.

4. The method of any of the preceding claims, wherein the first sensor data and the second sensor data each comprise low power sensor data.

5. The method of any of the preceding claims, wherein the first sensor data comprises inertial measurement unit (IMU) data and second sensor data comprises audio data.

6. The method of any of the preceding claims, wherein identifying the first data element comprises identifying an occurrence of motion of the user, and wherein identifying the second data element comprises identifying an occurrence of an audible sound.

7. The method of any of the preceding claims, wherein detecting the change in activity of the user comprises detecting a change in a motion type of the user.

8. The method of any of the preceding claims, wherein detecting the change in activity of the user comprises detecting a change in a pattern of motion of the user.

9. The method of any of the preceding claims, wherein detecting the change in activity of the user comprises detecting a change in an audible sound associated with the user.

10. The method of any of the preceding claims, wherein initiating the second process comprises initiating obtaining at least one image from an image sensor.

11. The method of any of the preceding claims, wherein initiating the second process comprises initiating computationally heavy process with respect to identifying the current activity of the user.

12. The method of claim 11, wherein the computationally heavy process comprises a large language model-based process.

13. The method of any of the preceding claims, wherein power requirements of utilizing the first process for a given length of time are less than power requirements of utilizing the second process for the given length of time.

14. A system comprising:
a processor;
a computer readable medium storing instructions that when executed by the processor cause the processor to perform operations comprising any one of the methods of claims 1-13.

15. A non-transitory computer-readable medium comprising instructions that when executed by a processor cause the processor to perform operations comprising any one of the methods of claims 1-13.
